# EUROPÄISCHE PATENTANMELDUNG

(11) **EP 1 843 080 A2**
(43) Veröffentlichungstag der Anmeldung: **10.10.2007**
(21) Anmeldenummer: 07004128.0
(22) Anmeldetag: 28.02.2007
(51) Int. Cl.: F21K 7/00, H01L 25/075

(54) **Halbleiter-Strahlungsquelle**

(30) Priorität: 03.04.2006 DE 102006015336
(71) Anmelder: Firma Ivoclar Vivadent AG, FL-9494 Schaan (LI)
(72) Erfinder: Plank, Wolfgang, A-6830 Rankweil (AT); Senn, Bruno, CH-9470 Buchs (CH)
(74) Vertreter: Baronetzky, Klaus

(57) **Zusammenfassung**

Die Erfindung betrifft eine Halbleiter-Strahlungsquelle (10), mit mindestens zwei Lichtquellen (16, 18), die auf einem gemeinsamen Basiskörper (14) befestigt sind und mit welchen gemeinsam Licht über ein Gesamt-Emissionsspektrum abgebbar ist, wobei eine erste Lichtquelle (16) ein kurzwelliges, insbesondere von 400 bis 430 nm, und eine zweite Lichtquelle (18) ein langwelligeres, insbesondere von etwa 450 bis 480 nm, Emissionsspektrum hat. Die erste Lichtquelle (16) ist in einer optischen Achse (22) angeordnet und die zweite Lichtquelle (18) weist mindestens zwei Chips (24, 26, 28, 30) auf, die, insbesondere symmetrisch zueinander und zur optischen Achse (22) und, die optische Achse (22) umgebend angeordnet sind.

## Beschreibung

Die Erfindung betrifft eine Halbleiter-Strahlungsquelle, gemäß dem Oberbegriff von Anspruch 1, ein Lichthärtgerät gemäß dem Oberbegriff von Anspruch 21 sowie eine Beleuchtungsvorrichtung gemäß dem Oberbegriff von Anspruch 24.

Eine derartige Ausbildung einer Strahlungsquelle unter Verwendung von Gruppen von Lichtquellen, die Licht unterschiedlicher Wellenlängen emittieren, ist seit längerem bekannt, beispielsweise aus der EP 0 151 686 A2 sowie der EP 879 582 A2. Bei der erstgenannten Lösung soll Dentalmaterial mit einer Wellenlänge zwischen 400 und 450 nm zunächst teilweise polymerisiert und anschließend mit einer Wellenlänge von 350 nm vollständig polymerisiert werden. Demgegenüber sollen bei der zweitgenannten Lösung LED-Chips mit verschiedenen Wellenlängen wie 435, 450 und 470 nm verwendet werden.

Die LEDs sind gebündelt angeordnet und werden gleichmäßig mit Spannung versorgt, so dass eine selektive Ansteuerung nicht möglich ist. Unterschiedlichen Photoinitiatoren mit unterschiedlicher spektraler Empfindlichkeit kann mit dieser Lösung nicht Rechnung getragen werden.

Es sind in den letzten 10 Jahren zahlreiche Untersuchungen unternommen wurden, um die Wirksamkeit der Photopolymerisation zu verbessern, um dem Zahnarzt oder ggf. dem Zahntechniker kürzere Behandlungszyklen zu ermöglichen, ohne dass die Sicherheit der dentalen Restauration bei der Durchhärtung gefährdet würde.

Dementsprechend sind verschiedene photopolymerisierbare Materialien untersucht und verwendet worden, wobei allgemein die Tendenz bestand, eine möglichst große spektrale Überlappung zwischen den Emissionsspektren der verwendeten Lichtquellen und den Empfindlichkeitsspektren der verwendeten Photoinitiatoren zu realisieren. Dies bedingt natürlich die Verwendung von unterschiedlichen spektralen Emissionsspektren, die bei unterschiedlichen Fotoinitiatoren angestrebt wurden.

In neuerer Zeit sind auch sogenannte dualhärtende Systeme vorgeschlagen wurden, wobei die Lösung gemäß der DE 101 25 340 A1 eine wesentliche Verbesserung des Härtergebnisses ermöglicht. Diese Lösung sieht zwei Halbleiter-Strahlungsquellen mit unterschiedlichen, voneinander beabstandeten Emissionsmaxima vor, die insbesondere bei unterschiedlichen Zeitpunkten erreicht werden.

Für die Bereitstellung der geforderten Lichtleistung werden bei dieser Lösung allerdings eine Vielzahl von LED-Chips verwendet, so dass diese Lösung eher für hochwertige Lichthärtgeräte in Betracht kommt. Um die gewünschte Härtung bereitzustellen, werden LED-Chips mit hoher Lichtemission, die zudem vergleichsweise teuer sind, verwendet, die eine intensive Lichtstrahlung abgeben. Dies umsomehr, als bei dieser Lösung die Härtung durch je eine Gruppe von LED-Chips nacheinander vorgenommen wird, so dass auch eine Untergruppe der LED-Chips eine für die Härtung ausreichende Leistung haben muss.

Demgegenüber liegt der Erfindung die Aufgabe zugrunde, eine Strahlungsquelle gemäß dem Oberbegriff von Anspruch 1 zu schaffen, die universell verwendbar ist, vergleichsweise preisgünstig herzustellen ist, aber dennoch eine gute Strahlungsausbeute ermöglicht.

Diese Aufgabe wird erfindungsgemäß durch Anspruch 1 gelöst. Vorteilhafte Weiterbildungen ergeben sich aus den Unteransprüchen.

Erfindungsgemäß ist es vorgesehen, dass die Lichtquellen in zwei Lichtquellen aufgeteilt sind, wobei beide Lichtquellen, die je aus mehreren LED-Chips bestehen können, in einem zentralen Bereich eines gemeinsamen Basiskörpers angeordnet sind. Die Lichtquellen nehmen damit nur einen recht kleinen Teil - beispielsweise 10 % oder 15 % oder gar nur 5 % - der Oberfläche des Basiskörpers ein.

Erfindungsgemäß ist es vorgesehen, dass die erste Lichtquelle in einer optischen Achse angeordnet ist und die zweite Lichtquelle mindestens zwei LED-Chips aufweist, die, insbesondere symmetrisch zueinander und zur optischen Achse und die optische Achse umgebend, angeordnet sind.

Überraschend führt dies zu einer ausgesprochenen hohen Lichtausbeute im Vergleich zu den bisherigen Lösungen. Durch den Verzicht auf eine Vielzahl von eher peripher angeordneten Lichtquellen lässt sich mit entsprechenden Sammellinsen die Lichtausbeute signifikant steigern. Dies bedeutet, dass bei gleicher eingeleiteter elektrischer Energie eine wesentlich höhere optische Strahlung abgebbar ist. Diesem Effekt kommt verstärkt zugute, dass bei Verzicht auf die peripheren LED-Chips ein im Verhältnis zur Chipfläche großer Basiskörper zur Verfügung steht, der dementsprechend als Kältepuffer wirkt. Die Wärmeableitung ist daher besonders hoch, gerade auch beim Impulsbetrieb, so dass die LED-Chips im Grunde im Volllastbereich oder gar im Überlastbereich gefahren werden können, ohne dass ihre Lebensdauer beeinträchtigt würde.

Erfindungsgemäß ist es vorgesehen, sämtliche LED-Chips nahe der optischen Achse anzuordnen. Eine erste Lichtquelle ist in der optischen Achse angeordnet, und eine zweite Lichtquelle diese umgebend und eng benachbart zu dieser.

Die zweite Lichtquelle ist bevorzugt symmetrisch angeordnet, wobei es sich versteht, dass für diese symmetrische Anordnung mindestens zwei LED-Chips erforderlich sind. Bevorzugt lässt sich die zweite Lichtquelle aus vier LED-Chips bilden, die je entlang einer Kante des quadratischen zentralen LED-Chips angeordnet sind, und zwar mit einem möglichst geringem Spalt zwischen den einzelnen LED-Chips.

Insgesamt ergibt sich so ein Kreuz aus der ersten Lichtquelle als zentralem LED-Chip und den vier LED-Chips der zweiten Lichtquelle, die an den LED-Chip der ersten Lichtquelle anschließend diesen umgeben.

Überraschend lässt sich hierdurch die Lichtausbeute wesentlich erhöhen, wobei es sich versteht, dass beliebige geeignete Mittel eingesetzt werden können, also beispielsweise eine Abdecklinse, die die LED-Chips abdeckt und das austretende Licht bündelt, so wie ggf. eine Sammellinse, die eine weitere Fokussierung der abgegebenen Lichtstrahlung ermöglicht.

Aufgrund der geringen Abmessungen der LED-Chips insgesamt ist aber auch direkte Beaufschlagung des Einleitendes eines Lichtleiters möglich.

Erfindungsgemäß besonders günstig ist es, dass durch die Aufteilung der Lichtabgabe auf zwei Lichtquellen Fotoinitiatoren in lichthärtenden Massen in beliebiger geeigneter Weise angeregt werden können. Beispielsweise kann die erste Lichtquelle auf die Anregung von Lucerin im Wellenlängenbereich von 400 bis 430 nm und die zweite Lichtquelle für die Anregung von Campherchinon für den Wellenlängenbereich zwischen 450 und 480 nm ausgelegt seien.

Bei Verwendung von Dentalmassen mit beiden Fotoinitiatoren können dann beide Lichtquellen gemeinsam eingeschaltet werden, während je nach dem verwendeten Material auch jede Lichtquelle getrennt eingeschaltet werden kann.

In besonders günstiger Ausgestaltung lässt sich die erfindungsgemäße Strahlungsquelle multifunktional verwenden, also nicht nur als Lichthärtgerät, sondern auch als Beleuchtungsgerät, insbesondere auch für die Prüfung, ob bei vorhandenen Kunststofffüllungen im Mund des Patienten Zahnspalten vorliegen oder nicht.

Hierfür ist ein Handgerät, das nach der Art eines Hand-Lichthärtgeräts geformt ist, besonders gut geeignet, denn durch den meist festen Lichtleiter und die schlanke Form kann die Lichtquelle gezielt unmittelbar in den Mund des Patienten eingeführt werden und dort die Beleuchtung blendfrei vorgenommen werden.

Bevorzugt wird zur Prüfung der Randspaltensituation lediglich eine der Lichtquellen, beispielsweise die erste, schwächere Lichtquelle eingeschaltet, damit keine Blendwirkung entsteht. In diesem Zusammenhang versteht es sich, dass auch die Prüf-Wellenlänge in weiten Bereichen an die Erfordernisse anpassbar ist; bevorzugt ist sie die kürzere Wellenlänge, die von der ersten, in der optischen Achse liegenden Lichtquelle abgegeben wird.

In einer weiteren vorteilhaften Ausgestaltung ist es vorgesehen, dass die zweite Lichtquelle vier LED-Chips aufweist, die nach der Art eines Kreuzes um die erste Lichtquelle angeordnet sind, und dass die erste Lichtquelle einen LED-Chip aufweist.

In einer weiteren vorteilhaften Ausgestaltung ist es vorgesehen, dass die Lichtquellen dicht aneinander angrenzen, wobei die Breite des zwischen ihnen verbleibenden Spalts weniger als ein Fünftel, insbesondere etwa ein Zehntel, des Durchmessers jedes LED-Chips beträgt. Die Spaltbreite beträgt bevorzugt 0,5 bis 2 mm und insbesondere etwa 1 mm.

In einer weiteren vorteilhaften Ausgestaltung ist es vorgesehen, dass die erste und die zweite Lichtquelle in einem zentralen Bereich des Basiskörpers angeordnet sind.

In einer weiteren vorteilhaften Ausgestaltung ist es vorgesehen, dass mindestens die erste Lichtquelle, insbesondere alle Lichtquellen auf einem zentralen Vorsprung, insbesondere mit einer Höhe zwischen 0,1 und 1 mm, des Basiskörpers angeordnet sind.

In einer weiteren vorteilhaften Ausgestaltung ist es vorgesehen, dass der Vorsprung in seiner Form den Lichtquellen folgt und insbesondere im Wesentlichen die Form eines Kreuzes aufweist.

In einer weiteren vorteilhaften Ausgestaltung ist es vorgesehen, dass die Lichtquellen auf dem Basiskörper oder dem Vorsprung durch eine Klebeverbindung oder durch eine Lötverbindung befestigt sind.

In einer weiteren vorteilhaften Ausgestaltung ist es vorgesehen, dass der Basiskörper und/oder der Vorsprung eine Wärmeleitfähigkeit aufweist, die besser als 0,5°C/W ist.

In einer weiteren vorteilhaften Ausgestaltung ist es vorgesehen, dass der Basiskörper und/oder der Vorsprung elektrisch leitfähig ist.

In einer weiteren vorteilhaften Ausgestaltung ist es vorgesehen, dass der Basiskörper wenigstens teilsweise aus Kupfer besteht.

In einer weiteren vorteilhaften Ausgestaltung ist es vorgesehen, dass der Basiskörper wenigstens teilweise mit Gold oder Nickel-Gold beschichtet ist.

In einer weiteren vorteilhaften Ausgestaltung ist es vorgesehen, dass einen zentralen Bereich insbesondere den Vorsprung, des Basiskörpers umgebend eine Printplatte angeordnet ist, die elektrische Anschlusskontakte trägt.

In einer weiteren vorteilhaften Ausgestaltung ist es vorgesehen, dass elektrische Anschlusskontakte für die Lichtquellen als Felder am Außenumfang des Basiskörper, insbesondere auf einer Printplatte, angeordnet sind, und dass sich Bonddrähte von den LED-Chips zu den elektrischen Anschlusskontakten erstrecken.

In einer weiteren vorteilhaften Ausgestaltung ist es vorgesehen, dass die Lichtquellen von einer konvexen Abdecklinse abgedeckt sind, die insbesondere auf der den Lichtquellen zugewandten Seite plan ausgebildet ist.

In einer weiteren vorteilhaften Ausgestaltung ist es vorgesehen, dass ein Abstandshalter, der insbesondere auf der Printplatte abgestützt ist, die Lichtquellen umgibt und dass der Abstandshalter insbesondere zusammen mit der Abdecklinse einen geschlossenen Raum vor den Lichtquellen bildet.

In einer weiteren vorteilhaften Ausgestaltung ist es vorgesehen, dass der geschlossene Raum eine flüssige oder viskose Substanz, insbesondere Silikongel, oder eine Vergußmasse aufweist.

In einer weiteren vorteilhaften Ausgestaltung ist es vorgesehen, dass ein optisches Element, das sich vor den Lichtquellen erstreckt, insbesondere die Abdecklinse oder eine Masse, die sich vor den Lichtquellen erstreckt, Phosphorpartikel aufweist.

In einer weiteren vorteilhaften Ausgestaltung ist es vorgesehen, dass vor den Lichtquellen, um ein mehrfaches deren Durchmesser beabstandet, insbesondere vor der Abdecklinse, eine Sammellinse angeordnet ist.

In einer weiteren vorteilhaften Ausgestaltung ist es vorgesehen, dass die Abdecklinse einen deutlich größeren Durchmesser als die Lichtquellen aufweist und eine Sammellinse einen deutlich größeren Durchmesser als die Abdecklinse aufweist, wobei die Durchmesserverhältnisse je insbesondere zwischen 1,2:1 und 10:1 liegen.

In einer weiteren vorteilhaften Ausgestaltung ist es vorgesehen, dass ein Lichtleiter in der optischen Achse der Halbleiter-Strahlungsquelle angeordnet ist, insbesondere in Strahlungsrichtung nach der Sammellinse.

In einer weiteren vorteilhaften Ausgestaltung ist es vorgesehen, dass die erste und zweite Lichtquelle gemeinsam einschaltbar sind.

In einer weiteren vorteilhaften Ausgestaltung ist es vorgesehen, dass die erste und zweite Lichtquelle zu unterschiedlichen Zeitpunkten einschaltbar bzw. ausschaltbar sind.

In einer weiteren vorteilhaften Ausgestaltung ist es vorgesehen, dass zur Beleuchtung und optischen Erfassung von Randspalten bei Kunststofffüllungen in oder an Zähnen, insbesondere über das Einschalten der ersten Lichtquelle.

Weitere Vorteile, Einzelheiten und Merkmale der Erfindung ergeben sich aus der nachstehenden Beschreibung mehrere Ausführungsbeispiele der Erfindung anhand der Zeichnung. Es zeigen:
- Fig. 1: eine erste schematische Ausführungsform einer erfindungs-gemäßen Strahlungsquelle in der Draufsicht;
- Fig. 2: eine zweite Ausführungsform einer erfindungsgemäßen Strahlungsquelle ebenfalls in der Draufsicht;
- Fig. 3: eine weitere Ausführungsform einer erfindungsgemäßen Strahlungsquelle in der Draufsicht;
- Fig. 4: die Ausführungsform gemäß Fig. 3 in der Seitenansicht;
- Fig. 5: eine weitere Ausführungsform einer erfindungsgemäßen Strahlungsquelle in der Draufsicht;
- Fig. 6: die Ausführungsform gemäß Fig. 5 in der Seitenansicht;
- Fig. 7: die Ausführungsform gemäß Fig. 5 und 6 in vergrößerter Darstellung;
- Fig. 8: eine weitere Ausführungform der erfindungsgemäßen Strahlungsquelle in der Draufsicht;
- Fig. 9: die Ausführungsform gemäß Fig. 8 im Seitenschnitt;
- Fig. 10: eine weitere Ausführungsform einer erfindungsgemäßen Strahlungsquelle in der Draufsicht;
- Fig. 11: die Ausführungsform gemäß Fig. 10 im seitlichen Schnitt;
- Fig. 12: eine weitere Ausführungsform einer erfindungsgemäßen Strahlungsquelle im seitlichen Schnitt; und
- Fig. 13: eine weitere Ausführungsform einer erfindungsgemäßen Strahlungsquelle im seitlichen Schnitt.

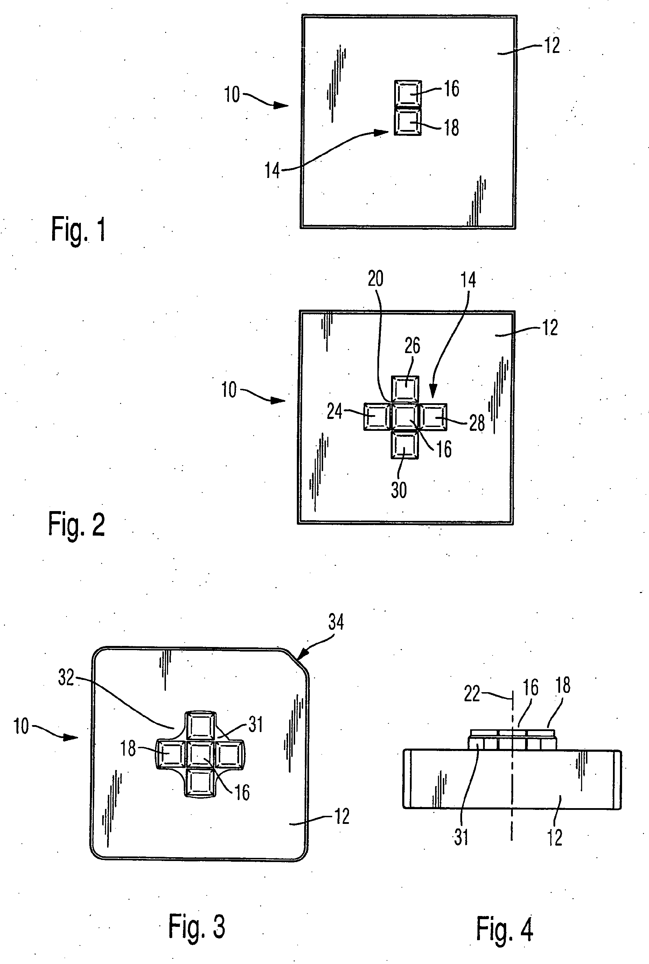

Fig. 1 zeigt eine Halbleiter-Strahlungsquelle 10 in schematischer Draufsicht. Es ist ein Basiskörper 12 vorgesehen, der in einem zentralen Bereich 14 zwei LED-Chips trägt. Hierbei ist eine erste Lichtquelle 16 vorgesehen, die in einer optischen Achse angeordnet ist. Beide Lichtquellen 16 und 18 sind in diesem Ausführungsbeispiel unmittelbar im zentralen Bereich des Basiskörpers 12 angeordnet, auf dem sie gelagert sind, und der zugleich der Abführung der von den LED-Chips erzeugten Wärme dient. In diesem Ausführungbeispiel sind die beiden LED-Chips je unmittelbar angrenzend an die optische Achse 22 angeordnet. Sie weisen einen Spalt 20 zwischen sich auf, der so gering gehalten ist, wie es technisch und elektrisch möglich ist, um Kurzschlüsse zu vermeiden, wobei der Abstand wenige Mikrometer betragen kann.

Jeder LED-Chip ist in an sich bekannter Weise quadratisch ausgebildet.

Aus Fig. 2 ist eine weitere Ausführungsform der erfindungsgemäßen Halbleiter-Strahlungsquelle 10 ersichtlich. Hier wie auch in den weiteren Figuren weisen gleiche Bezugszeichen auf gleiche oder entsprechende Elemente hin. In dem Ausführungsbeispiel gemäß Fig. 2 ist die erste Lichtquelle 16 unmittelbar in der optischen Achse 22 vorgesehen. Die zweite Lichtquelle 18 besteht aus vier LED-Chips 24, 26, 28 und 30, die kreuzförmig um den LED-Chip der ersten Lichtquelle 18 herum angeordnet sind. Alle Chips weisen die gleichen Abmessungen auf, so dass die Kantenlängen einander entsprechen. Es sind wiederum ausgesprochen schmale Spalten 20 vorgesehen, die eine elektrische Trennung ermöglichen, jedoch die räumlich Nähe sämtlicher LED-Chips im zentralen Bereich 14 nicht beeinflussen.

Auch hier sind die erste und die zweite Lichtquelle 16 und 18 unabhängig voneinander schaltbar und nehmen den zentralen Bereich 14 des Basiskörpers 12 ein, wobei ein wesentlich größerer Bereich frei bleibt.

Aus Fig. 3 ist eine weitere Ausführungsform einer erfindungsgemäßen Strahlungsquelle 10 ersichtlich. Bei dieser Ausführungsform, die in Fig. 4 in Seitenansicht dargestellt ist, ist ein Vorsprung 31 vorgesehen, der die erste und die zweite Lichtquelle 16 und 18 trägt. Der Vorsprung 31 folgt in seiner Form dem durch die erste und zweite Lichtquelle 16 und 18 gebildeten Kreuz, wobei die Übergänge der Schenkel bei 32 je Radien aufweisen und insofern etwas ausgerundet und vergrößert sind. Dies ermöglicht eine vereinfachte Montage und eine gute Wärmeableitung der LED-Chips auf den Basiskörper 10.

Der Basiskörper 10 besteht bevorzugt im Wesentlichen aus Kupfer und ist insbesondere an seiner Vorderseite, also den Lichtquellen benachbart, mit einer Nickel-Gold-Schicht beschichtet. In modifizierter Ausgestaltung ist der Basiskörper außen vollständig mit dieser Schicht beschichtet.

Aus Fig. 3 ist ersichtlich, dass der Basiskörper 12 eine Abschrägung 34 an einer Ecke aufweist. Die Abschrägung 34 dient der Montageerleichterung, damit sichergestellt ist, dass die anhand der folgenden Figuren erörterten Kontaktflächen zutreffend geschaltet werden.

Aus Fig. 4 ist ersichtlich, dass die Stärke der LED-Chips wesentlich geringer als die Stärke des Basiskörpers ist, beispielsweise um den Faktor 10.

Zudem ist die Stärke der LED-Chips für die Lichtquellen 16 und 18 auch etwas geringer als die Höhe des Vorsprungs 31.

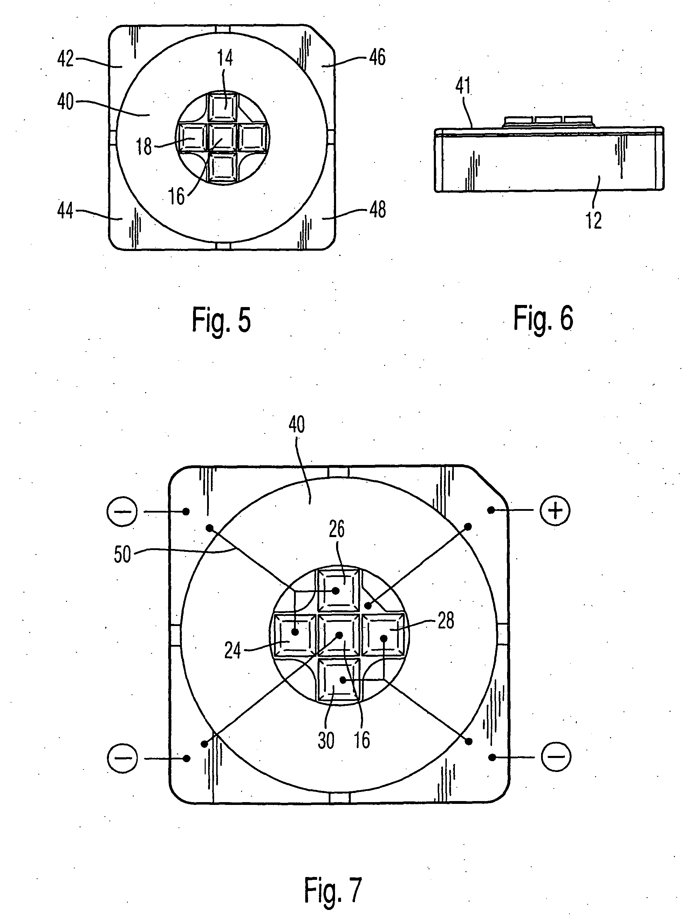

Fig. 5 zeigt, dass der zentrale Bereich 14 von einer Ringfläche 40 umgeben sein kann, die bevorzugt durch eine auf dem Basiskörper 12 aufliegenden Printplatte 41 gebildet ist. Bevorzugt ist ein Kreisring ausgebildet, der in die im Wesentlichen quadratische Form des Basiskörpers 12 eingepasst ist.

Besonders günstig ist es, dass jenseits der Ringfläche 40 vier voneinander beabstandete Kontaktfelder 42, 44, 46 und 48 gebildet sind. Die Kontaktfelder 42 bis 48 dienen der Kontaktierung von Anschlussdrähten oder Bonddrähten für die LED-Chips der Lichtquellen 16, 18. Demnach erstrecken sich die Bonddrähte (vgl. auch Fig. 7) über die Ringfläche 40 hinweg. Auch dies kommt der Konzentrierung der Lichtabgabe auf den wirklich wichtigen zentralen Bereich 14 zugute.

Aus Fig. 6 ist ersichtlich, dass sich die Printplatte 41 im Wesentlichen in gleicher Höhe wie der Vorsprung 31 erstrecken kann. Es versteht sich, dass in beliebiger Weise eine Höhenanpassung vorgenommen werden kann, so dass auch die Printplatte oder der Vorsprung dicker sein können.

Bei dieser Ausführungsform sind zusätzlich Vorwiderstände für die LED-Chips vorgesehen. Mit diesen Vorwiderständen lässt sich bei Parallelschaltung von LED-Chips eine Kalibrierung vornehmen, so dass auch unsortierte LED-Chips, die preisgünstig sind, eingesetzt werden können.

Aus Fig. 7 ist ersichtlich, in welcher Weise sich die Bonddrähte 50 zur Kontaktierung der einzelnen LED-Chips erstrecken. Durch die entsprechende Kontaktgabe ist eine separate Ansteuerung der LED-Chips 24 bis 30 einerseits und der Lichtquelle 16 andererseits gewährleistet.

Es versteht sich, dass die elektrische Isolierung der LED-Chips 24 bis 30 sowie des LED-Chips der Lichtquelle 16 in an sich bekannter Weise realisiert sein kann, beispielsweise durch eine entsprechende Oxidationsschicht des verwendeten Halbleitermaterials. Dem steht nicht entgegen, dass die LED-Chips sicher auf dem Vorsprung 31 oder dem Basiskörper 12 befestigt sein können.

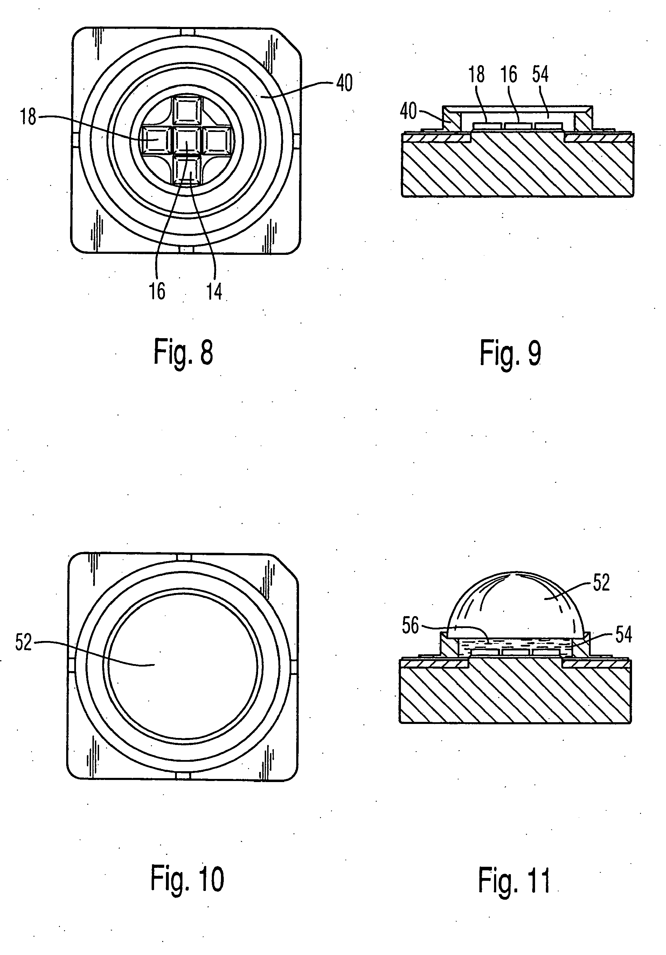

Aus Fig. 7 ist ersichtlich, dass ein Abstandshalter 40 den zentralen Bereich 14 umgeben kann. Der Abstandhalter 40 kann beispielsweise aus Kunststoff oder Leichtmetall gebildet sein und die Lichtquellen 16 und 18 schützen. In dem dargestellten Ausführungsbeispiel ist er ringförmig und ist für die Aufnahme der aus Fig. 10 und 11 ersichtlichen Abdecklinse 52 vorgesehen.

Während bei der Ausführungform gemäß den Fig. 8 und 9 ein Raum 54 oberhalb der Lichtquellen 16 und 18 verbleibt, ist dieser Raum 54 bei der Ausführungsform gemäß Fig. 10 und 11 geschlossen und mit einer besonderen Substanz gefüllt. In dem dargestellten Ausführungsbeispiel ist hierfür Silikongel 56 vorgesehen, das mit gelben Phosphorpartikel versehen ist. Hierdurch ist sichergestellt, dass das abgegebene Licht einen höheren Weißanteil erhält, ohne dass die Lichtleistung nennenswert beeinträchtigt werden würde.

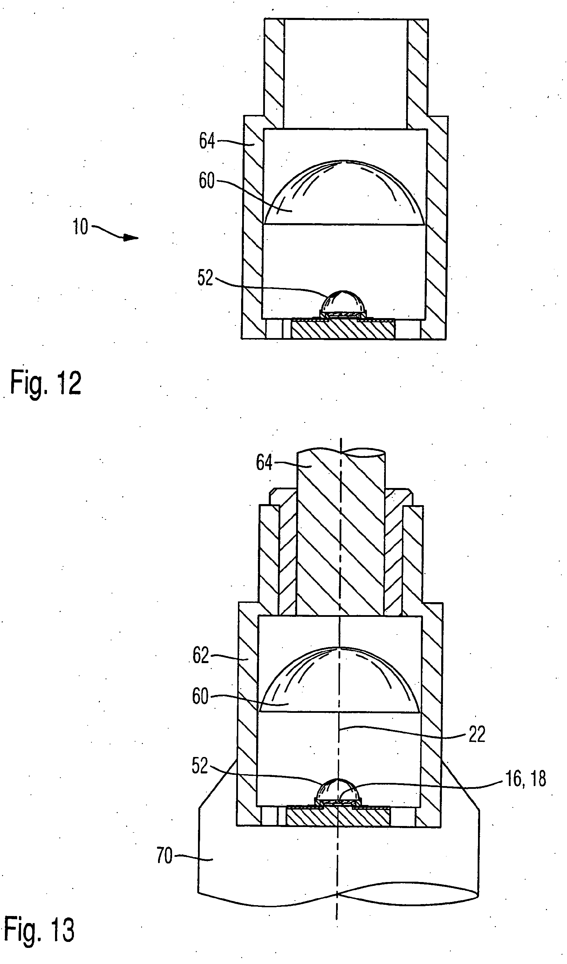

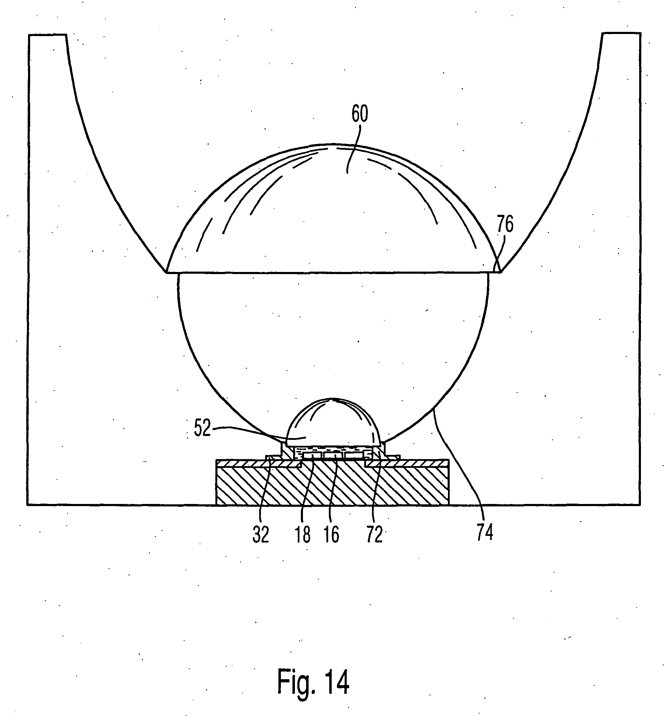

In einer besonders bevorzugten Ausgestaltung, die aus Fig. 12 und in modifizierter Form aus Fig. 13 ersichtlich ist, ist die erfindungsgemäße Strahlungsquelle in ein partiell dargestelltes kombiniertes Beleuchtungs- und Lichthärtgerät eingebaut. Hierzu ist eine Sammellinse 60 vorgesehen, die über einen Optikhalter 62 so gelagert ist, dass sie sich oberhalb der Abdecklinse 52 erstreckt und zwar diese überlappend und abdeckend. In der Ausführungsform gemäß Fig. 13 erstreckt sich ein Lichtleiter 64 in Strahlungsrichtung anschließend an die Sammellinse 60. Das Lichthärtgerät weist, wie es in Fig. 13 schematisch angedeutet ist, ein Gehäuse 70 auf und ist als Handgerät ausgebildet.

Wie aus den Fig. 12 und 13 ersichtlich ist, sind sowohl die Lichtquellen 16, 18 als auch die Abdecklinse 52, aber auch die Sammellinse 60 und der Lichtleiter 64 entlang der optischen Achse 22 angeordnet. Dies ermöglicht eine besonders gute Ausbeute und leichte Fokussierung auf den gewünschten Brennpunkt.

## Patentansprüche

1. Halbleiter-Strahlungsquelle, mit mindestens zwei Lichtquellen, die auf einem gemeinsamen Basiskörper befestigt sind und mit welchen gemeinsam Licht über ein Gesamt-Emissionsspektrum abgebbar ist, wobei eine erste Lichtquelle ein kurzwelliges, insbesondere von 400 bis 430 nm, und eine zweite Lichtquelle ein langwelligeres, insbesondere von etwa 450 bis 480 nm, Emissionsspektrum hat, **dadurch gekennzeichnet, dass** eine der Lichtquellen (16), insbesondere die erste, in einer optischen Achse (22) angeordnet ist und die andere Lichtquelle (18), insbesondere die zweite, mindestens zwei LED-Chips (24, 26, 28, 30) aufweist, die, insbesondere symmetrisch zueinander und zur optischen Achse (22) und die optische Achse (22) umgebend, angeordnet sind.

2. Strahlungsquelle nach Anspruch 1, **dadurch gekennzeichnet, dass** die zweite Lichtquelle (18) vier LED-Chips (24, 26, 28, 30) aufweist, die nach der Art eines Kreuzes oder Sterns um die erste Lichtquelle (16) angeordnet sind, und dass die erste Lichtquelle (16) einen LED-Chip aufweist.

3. Strahlungsquelle nach einem der vorhergehenden Ansprüche, **dadurch gekennzeichnet, dass** die Lichtquellen (16, 18) dicht aneinander angrenzen.

4. Strahlungsquelle nach einem der vorhergehenden Ansprüche, **dadurch gekennzeichnet, dass** alle LED-Chips (16, 24, 26, 28, 30) in einem zentralen Bereich (14) des Basiskörpers (12) angeordnet sind.

5. Strahlungsquelle nach einem der vorhergehenden Ansprüche, **dadurch gekennzeichnet, dass** die Lichtquellen auf dem Basiskörper (12) durch eine Klebeverbindung oder durch eine Lötverbindung befestigt sind.

6. Strahlungsquelle nach einem der vorhergehenden Ansprüche, **dadurch gekennzeichnet, dass** der Basiskörper (12) eine Wärmeleitfähigkeit aufweist, die besser als 0,5°C/W ist.

7. Strahlungsquelle nach einem der vorhergehenden Ansprüche, **dadurch gekennzeichnet, dass** sich zwischen den LED-Chips (24, 26, 28, 30), insbesondere zwischen äußeren LED-Chips, und der Printplatte (41) Strahlungsabsorber erstrecken, die insbesondere mit dem Basiskörper (22) in Wärmeleitverbindungen miteinander verbunden sind.

8. Strahlungsquelle nach Anspruch 7, **dadurch gekennzeichnet, dass** die Strahlungsabsorber zugleich wärmedämmend ausgebildet sind und insbesondere aus Keramik bestehen.

9. Strahlungsquelle nach einem der Ansprüche 7 oder 8, **dadurch gekennzeichnet, dass** die Strahlungsabsorder sich mindestens über die Breite der LED-Chips (16, 24, 26, 28, 30) erstrecken und insbesondere eine größere Höhe als die LED-Chips (24, 26, 28, 30) aufweisen, bevorzugt etwa die 1,5- bis 5-fache Höhe und besonders bevorzugt etwa die zweifache Höhe der LED-Chips (24, 26, 28, 30).

10. Strahlungsquelle nach einem der vorhergehenden Ansprüche, **dadurch gekennzeichnet, dass** der Basiskörper (12) elektrisch leitfähig ist.

11. Strahlungsquelle nach einem der vorhergehenden Ansprüche, **dadurch gekennzeichnet, dass** der Basiskörper (12) wenigstens teilsweise aus Kupfer besteht und insbesondere wenigstens teilweise mit Gold oder Nickel-Gold beschichtet ist.

12. Strahlungsquelle nach Anspruch 10 oder 11, **dadurch gekennzeichnet, dass** wenigstens ein mit einem LED-Chip (24, 26, 28, 30) zusammenwirkender Vorwiderstand (49) auf der Printplatte (41) außerhalb des Abstandshalters (40) frei zugänglich angeordnet ist.

13. Strahlungsquelle nach Anspruch 12, **dadurch gekennzeichnet, dass** der Vorwiderstand (49) abgleichbar ist.

14. Strahlungsquelle nach einem der vorhergehenden Ansprüche, **dadurch gekennzeichnet, dass** elektrische Anschlussflächen für die Lichtquellen als Felder, insbesondere auf einer Printplatte (41) angeordnet sind, und dass sich Bonddrähte (50) von den LED-Chips (24, 26, 28, 30) zu den elektrischen Anschlussflächen erstrecken.

15. Strahlungsquelle nach einem der vorhergehenden Ansprüche, **dadurch gekennzeichnet, dass** mindestens zwischen zwei einander benachbarten LED-Chips (16, 24, 26, 28, 30) ein Reflektorelement angeordnet ist, dass sich an dem Basiskörper (12) abstützt.

16. Stahlungsquelle nach einem der vorhergehenden Ansprüche, **dadurch gekennzeichnet, dass** das sich zwischen zwei LED-Chips (24, 30) erstreckende Reflektorelement zwei im Wesentlichen schräg verlaufende Reflexionsflächen aufweist, wobei jede Reflexionsfläche aus dem benachbartem LED-Chip stammende Strahlung reflektiert.

17. Strahlungsquelle nach einem der vorhergehenden Ansprüche, **dadurch gekennzeichnet, dass** Reflexionsflächen leicht konkav oder parabolisch ausgestaltet sind und dass das Reflektorelement (2) einem im Wesentlichen dreieckigen Querschnitt, insbesondere im Wesentlichen eines gleichschenkligen Dreiecks, aufweist.

18. Strahlungsquelle nach einem der vorhergehenden Ansprüche, **dadurch gekennzeichnet, dass** die Reflexionsflächen in Richtung der optischen Achse betrachtet sich im Wesentlichen entsprechend der Höhe wenigstens einer auf dem Basiskörper (12) gelagerten Printplatte (41) erstrecken oder diese überragen.

19. Strahlungsquelle nach einem der vorhergehenden Ansprüche, **dadurch gekennzeichnet, dass** mehrere Reflektorelemente unter Bildung eines Gitterreflektors miteinander verbunden sind und dass sich insbesondere der Gitterreflektor an dem Basiskörper und/oder einer Printplatte (41) und/oder den LED-Chips abstützt.

20. Strahlungsquelle nach einem der vorhergehenden Ansprüche, **dadurch gekennzeichnet, dass** sich die Printplatte (41) von den zentral angeordneten LED-Chips (24, 26, 28, 30) nach außen, insbesondere zum Umfangsbereich des Basiskörpers (12) erstreckt.

21. Strahlungsquelle nach einem der vorhergehenden Ansprüche, **dadurch gekennzeichnet, dass** die Printplatte (41) im Wesentlichen die gleiche Höhe aufweist wie die LED-Chips.

22. Strahlungsquelle nach einem der vorhergehenden Ansprüche, **dadurch gekennzeichnet, dass** insbesondere über Bondverbindungen Anschlussflächen von Leiterbahnen der Printplatte (41) mit den LED-Chips (16, 24, 26, 28, 30) verbunden sind.

23. Strahlungsquelle nach einem der vorhergehenden Ansprüche, **dadurch gekennzeichnet, dass** die Printplatte (41) eine Epoxidharzbasis aufweist und wenigstens auf einer Seite mindestens eine Leiterbahn aufweist und insbesonder mit Kupfer beschichtet sowie durchkontaktiert ist.

24. Strahlungsquelle nach einem der vorhergehenden Ansprüche, **dadurch gekennzeichnet, dass** eine konvexe Abdecklinse im Strahlengang nach den LED-Chips (16, 24, 26, 28, 30) angeordnet ist, die insbesondere auf der den Lichtquellen zugewandten Seite plan ausgebildet ist, und ein Abstandshalter für diese im Wesentlichen rohr- oder ringförmig ausgebildet ist und dass der Abstandshalter mindestens teilweise auf der Printplatte (41) und/oder dem Basiskörper (12) abgestützt ist.

25. Strahlungsquelle nach einem der vorhergehenden Ansprüche, **dadurch gekennzeichnet, dass** mindestens eine Leiterbahn der Printplatte (41) unter einem Abstandshalter hindurch verläuft und dass der Abstandshalter insbesondere gegen die Leiterbahn abgedichtet ist.

26. Strahlungsquelle nach einem der vorhergehenden Ansprüche, **dadurch gekennzeichnet, dass** sich zwischen den LED-Chips (16, 24, 26, 28, 30), dem Abstandshalter und der Abdeckleiste ein geschlossener Raum erstreckt, der eine durchsichtige oder durchscheinende, flüssige oder gelförmige Substanz, insbesondere Silikongel oder eine Vergussmasse ausweist.

27. Strahlungsquelle nach einem der vorhergehdenen Ansprüche, **dadurch gekennzeichnet, dass** die Substanz Phosphorpartikel aufweist.

28. Strahlungsquelle nach einem der vorhergeheden Ansprüche, **dadurch gekennzeichnet, dass** im Strahlengang nach einer Abdecklinse eine Sammellinse angeordnet ist, deren Durchmesser insbesondere größer als der Durchmesser der Abdecklinse ist.

29. Strahlungsquelle nach einem der vorhergehenden Ansprüche, **dadurch gekennzeichnet, dass** von den LED-Chips (16, 24, 26, 28, 30) beabstandet vor diesen, also im Strahlengang nach diesen und insbesondere auch im Strahlengang nach einer Abdecklinse ein Reflektor angeordnet ist.

30. Strahlungsquelle nach einem der vorhergehenden Ansprüche, **dadurch gekennzeichnet, dass** im Strahlengang nach der Sammellinse oder nach dem Reflektor ein Lichtleiter angeordndet ist.

31. Strahlungsquelle nach einem der vorhergehenden Ansprüche, **dadurch gekennzeichnet, dass** die Abdecklinse (52) einen deutlich größeren Durchmesser als die Lichtquellen (16, 18) aufweist und eine Sammellinse (60) einen deutlich größeren Durchmesser als die Abdecklinse (52) aufweist, wobei die Durchmesserverhältnisse je insbesondere zwischen 1,2:1 und 10:1 liegen.

32. Strahlungsquelle nach einem der vorhergehenden Ansprüche, **dadurch gekennzeichnet, dass** die erste und die zweite Lichtquelle gemeinsam oder zu unterschiedlichen Zeitpunkten einschaltbar und/oder gemeinsam oder zu unterschiedlichen Zeitpunkten ausschaltbar sind.

33. Lichthärtgerät, insbesondere handgeführtes, mit einer Strahlungsquelle (10) gemäß einem der vorhergehenden Ansprüche, insbesondere zum Härten von lichtpolymerisierbaren Dentalmassen.

34. Beleuchtungsgerät, insbesondere handgeführtes, mit einer Strahlungquelle nach einem der Ansprüche 1 bis 32, bei dem mindestens die erste Lichtquelle (16) eingeschaltet ist.

35. Verwendung eines Beleuchtungsgeräts nach Anspruch 34, zur Beleuchtung und optischen Erfassung von Randspalten bei Kunststofffüllungen in oder an Zähnen, insbesondere über das Einschalten der ersten Lichtquelle (16).
